Europäisches Patentamt

European Patent Office

Office européen des brevets

(11) Publication number: **0 148 311**
**B1**

# EUROPEAN PATENT SPECIFICATION

(45) Date of publication of patent specification: 20.07.88

(21) Application number: 84105149.3

(22) Date of filing: 07.05.84

(51) Int. Cl.⁴: **C 07 K 7/10,** C 12 P 19/34,
C 12 N 15/00, C 12 P 21/00,
A 61 K 37/02 // C12R1:19

(54) A novel physiologically active polypeptide.

(30) Priority: 26.12.83 JP 251817/83
08.02.84 JP 19850/84
18.04.84 JP 76584/84

(43) Date of publication of application:
17.07.85 Bulletin 85/29

(45) Publication of the grant of the patent:
20.07.88 Bulletin 88/29

(84) Designated Contracting States:
DE FR GB

(56) References cited:
EP-A-0 012 494

THE BRITISH JOURNAL OF CANCER, vol. 44,
no. 3, September 1981, London,N. MATTHEWS
"Tumor-Necrosis Factor from the Rabbit. V.
Synthesis in Vitro by Mononuclear Phagocytes
from Various Tissues of Normal and BCG-
Injected Rabbits", pages 418-424

(73) Proprietor: Asahi Kasei Kogyo Kabushiki Kaisha
2-6, Dojimahama 1-chome Kita-ku
Osaka-shi Osaka 530 (JP)

(72) Inventor: Itoh, Hirataka
490-341, Nakano
Fuji-shi Shizuoka-ken (JP)

(74) Representative: Boeters, Hans Dietrich, Dr. et al
Boeters, Bauer & Partner Thomas-Wimmer-Ring
14
D-8000 München 22 (DE)

(56) References cited:
THE BRITISH JOURNAL OF CANCER, vol. 42,
no. 3, September 1980, London,N. MATTHEWS
et al. "Tumor-Necrosis Factor from the Rabbit.
IV. Purification and Chemical
Characterization", pages 416-422

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a deoxyribonucleic acid (hereinafter referred to as "DNA") coding for a novel physiologically active polypeptide. This invention also relates to a replicable recombinant DNA containing the DNA, a microorganism or cell transformed with the replicable recombinant DNA, a novel physiologically active polypeptide obtained by expressing the DNA. The present invention is also concerned with a process for producing the physiologically active polypeptide. The present invention is further concerned with a substantially pure physiologically active polypeptide having the amino acid sequence described herein, and pharmaceutical compositions containing the physiologically active polypeptide as the effective ingredient. More particularly, the present invention is concerned with a DNA coding for TNF (Tumor Necrosis Factor), TNF having an amino acid sequence deduced from the base sequence of the DNA, a process for producing TNF from the DNA utilizing recombinant DNA technology, and the use of the product obtained by the process.

In the present specification, amino acids and peptides are represented using abbreviations, as indicated below, approved by IUPAC-IUB Commission on Biochemical Nomenclature (CBN). Incidentally, with respect to amino acids and the like having isomers, those represented by the following abbreviations are of the L-configuration unless otherwise specified.

| | |
|---|---|
| Gln: | glutamine residue |
| Asp: | aspartic acid residue |
| Pro: | proline residue |
| Tyr: | tyrosine residue |
| Val: | valine residue |
| Lys: | lysine residue |
| Glu: | glutamic acid residue |
| Ala: | alanine residue |
| Asn: | Asparagine residue |
| Leu: | leucine residue |
| Phe: | phenylalanine residue |
| Gly: | glycine residue |
| His: | histidine residue |
| Ser: | serine residue |
| Thr: | threonine residue |
| Ile: | isoleucine residue |
| Trp: | tryptophan residue |
| Arg: | arginine residue |
| Met: | methionine residue |
| Cys: | cysteine residue |

Polydeoxyribonucleotides and oligodeoxyribonucleotides are represented by sequences of deoxyribonucleotide residue which are abbreviated as follows:

| | |
|---|---|
| A: | 2'-deoxyadenylic acid residue |
| C: | 2'-deoxycytidylic acid residue |
| G: | 2'-deoxyguanylic acid residue |
| T: | thymidylic acid residue |

Unless otherwise specified, the left end of the sequence of deoxynucleotides is the 5' end.

There are known various substances having a capacity for stimulating the reticuloendothelial system, for example, physiologically active substances having antitumor activity which are induced by various Gram-positive bacteria and endotoxins. Specifically, Carswell et al discovered that the serum from CD-1 Swiss mice infected with bacillus Calmette-Guérin (BCG), and after two weeks, followed by intravenous injection of endotoxin has cytotoxic activity against cultured L cells and also discovered a phenomenon that it induces hemorrhagic necrosis of transplanted Meth A sarcoma in the (BALB/c×C57BL/6)F$_1$ mouse. They gave the name of TNF (Tumor Necrosis Factor) to the active substance in the serum [Proc. Nat. Acad. Sci. USA, Vol. 72 (No. 9), pp. 3666—3670 (1975)]. Thereafter, Ruff et al reported that the rabbit TNF prepared according to the above-mentioned method proposed by Carswell et al was purified about 2,000-fold over the serum [J. Immunol., Vol. 125 (No. 4), pp. 1671—1677 (1980)]. Further, Matthews et al reported that the rabbit TNF was purified about 1,000-fold over the serum [Br. J. Cancer, Vol. 42, pp. 416—422 (1980)]. However, in Ruff et al and Matthews et al, the tumor necrosis effect with respect to the purified TNF is not confirmed in animal experiments.

Japanese Patent Application Laid-Open Specification No. 57—140725 (1982) discloses a process for isolating and purifying a proteinaceous physiologically active substance having antitumor activity, which is induced by administering to a mammal such as mouse, rabbit or guinea pig at least one substance having a capacity for stimulating the reticuloendothelial system and then injecting endotoxin from a Gram-negative bacterium into the mammal, or by adding endotoxin from a Gram-negative bacterium to a tissue culture containing activated macrophages from a mammal. In this Japanese Patent Application Laid-Open Specification, there are also disclosed the molecular weight and isoelectric point of the purified proteinaceous physiologically active substance (molecular weight, 39,000±5,000 as measured by gel filtration and SDS-polyacrylamide gel electrophoresis; isoelectric point, pH 3.9±0.3 as measured by isoelectric focusing) but not any detailed structure of the proteinaceous physiologically active substance.

Meanwhile, Matthews reported that there is obtained a substance having cytotoxic activity against L cells by a process in which BCG is injected into a rabbit and mononuclear phagocytes from various tissues of the rabbit are obtained two weeks after the injection, followed by addition of endotoxin to the cell culture of the mononuclear phagocytes [Br. J. Cancer, Vol. 44 (3), pp. 418—424 (1981)]. However, in his report, the detailed structure of the obtained substance is not disclosed and, further, there is no evidences showing that the obtained substance is identical with TNF found in the serum.

The present inventor has made extensive and intensive studies on properties and structure of rabbit TNF and rabbit-TNF producing cells. As a result, he obtained cells capable of producing a substance having cytotoxic activity against L cells by administering a substance having a capacity for stimulating the reticuloendothelial system to a rabbit, followed by injection of endotoxin derived from a bacterium into the rabbit, and then obtained such a substance using the L cells. He also affirmed that the molecular weight and immunological properties of the substance having cytotoxic activity against L cells obtained using the above obtained cells are in agreement with those of TNF obtained from rabbit serum. Meanwhile, with the progress of genetic manipulation techniques, it became possible to determine the structrue of a protein so long as a DNA coding for the protein is obtained in isolated form. This is so because the structure of the isolated DNA can be determined and, then, the structure of the protein can be deduced from the structure of the DNA. Further, it became possible to produce a protein from a DNA coding for the protein utilizing a microorganism or cell culture. The present inventor applied the above-mentioned genetic manipulation techniques to the cells capable of producing a substance having cytotoxic activity against L cells. As a result, he has surprisingly found that a pure DNA coding for a physiologically active polypeptide can be skillfully isolated and the structure of the DNA and that of the physiologically active polypeptide can be determined, and that the physiologically active polypeptide can be obtained in substantially pure form using the DNA.

The present invention has been made based on such novel findings.

Therefore, it is an object of the present invention to provide a physiologically active polypeptide.

It is another object of the present invention to provide a DNA coding for TNF.

It is still another object of the present invention to provide a replicable recombinant DNA comprising a DNA coding for TNF and a replicable expression vehicle.

It is a further object of the present invention to provide a microorganism or a cell transformed with a recombinant DNA of the kind as mentioned above.

It is still a further object of the present invention to provide a process for producing a physiologically active polypeptide of the kind as mentioned above.

The foregoing and other objects, features and advantages of the present invention will be apparent from the following detailed description taken in connection with the accompanying drawings in which:

Fig. 1 illustrates the restriction maps of plasmids each containing a DNA coding for a rabbit physiologically active polypeptide of the present invention;

Fig. 2 illustrates the flow-sheet of the method for the preparation of a recombinant DNA (pTNF-lac-1) coding for the rabbit physiologically active polypeptide of the present invention; and

Fig. 3 illustrates the flow-sheet of the method for the preparation of another recombinant DNA (pTNF-lacUV5-1) coding for the rabbit physiologically active polypeptide of the present invention.

Essentially, according to the present invention, there is provided a physiologically active polypeptide having an amino acid sequence represented by the following formula (I):

| Ser | Ala | Ser | Arg | Ala | Leu | Ser | Asp | Lys | Pro | Leu | Ala | His | Val | Val |
|-----|-----|-----|-----|-----|-----|-----|-----|-----|-----|-----|-----|-----|-----|-----|
| Ala | Asn | Pro | Gln | Val | Glu | Gly | Gln | Leu | Gln | Trp | Leu | Ser | Gln | Arg |
| Ala | Asn | Ala | Leu | Leu | Ala | Asn | Gly | Met | Lys | Leu | Thr | Asp | Asn | Gln |
| Leu | Val | Val | Pro | Ala | Asp | Gly | Leu | Tyr | Leu | Ile | Tyr | Ser | Gln | Val |
| Leu | Phe | Ser | Gly | Gln | Gly | Cys | Arg | Ser | Tyr | Val | Leu | Leu | Thr | His |
| Thr | Val | Ser | Arg | Phe | Ala | Val | Ser | Tyr | Pro | Asn | Lys | Val | Asn | Leu |
| Leu | Ser | Ala | Ile | Lys | Ser | Pro | Cys | His | Arg | Glu | Thr | Pro | Glu | Glu |
| Ala | Glu | Pro | Met | Ala | Trp | Tyr | Glu | Pro | Ile | Tyr | Leu | Gly | Gly | Val |
| Phe | Gln | Leu | Glu | Lys | Gly | Asp | Arg | Leu | Ser | Thr | Glu | Val | Asn | Gln |
| Pro | Glu | Tyr | Leu | Asp | Leu | Ala | Glu | Ser | Gly | Gln | Val | Tyr | Phe | Gly |
| Ile | Ile | Ala | Leu | | | | | | | | | | | |

wherein Gln stands for a glutamine residue, Asp an aspartic acid residue, Pro a proline residue, Tyr a tyrosine residue, Val a valine residue, Lys a lysine residue, Glu a glutamic acid residue, Ala an alanine residue, Asn an asparagine residue, Leu a leucine residue, Phe a phenylalanine residue, Gly a glycine residue, His a histidine residue, Ser a serine residue, Thr a threonine residue, Ile an isoleucine residue, Trp a

**0 148 311**

tryptophan residue, Arg an arginine residue, Met a methionine residue, and Cys a cysteine residue.

The rabbit TNF of the present invention includes a polypeptide having an amino acid methionine attached to the N-terminum of the above-mentioned amino acid sequence and an intermediate in which a partial or entire peptide deduced from a DNA having a base sequence represented by the following formula (III):

```
ATGAGCACTGAGAGTATGATCCGGGACGTCGAGCTGGCGGAGGGGCCGCTCCCCAAGAAGG
CAGGGGGGCCCCAGGGCTCCAAGCGTTGCCTCTGCCTCAGCCTCTTCTCTTTCCTGCTCGT
GGCTGGAGCCACCACGCTCTTCTGCCTGCTGCACTTCAGGGTGATCGGCCCTCAGGAGGAA
GAGCAGTCCCCAAACAACCTCCATCTAGTCAACCCTGTGGCCCAGATGGTCACCCTCAGA
```

is attached to the N-terminus of the above-mentioned amino acid sequence. It is possible to change part of the structure of a DNA coding for a polypeptide by natural or artificial mutation without no significant change of the activity of the polypeptide. The rabbit TNF of the present invention includes a polypeptide having a structure corresponding to homologuus variant of the polypeptide having the abovementioned amino acid sequence. All such physiologically active polypeptides are hereinafter referred to as "TNF".

In another aspect of the present invention, there is provided a deoxyribonucleic acid comprising a base sequence coding for a physiologically active polypeptide,

said physiologically active polypeptide having an amino acid sequence represented by the following formula (I):

| | | | | | | | | | | | | | | |
|---|---|---|---|---|---|---|---|---|---|---|---|---|---|---|
| Ser | Ala | Ser | Arg | Ala | Leu | Ser | Asp | Lys | Pro | Leu | Ala | His | Val | Val |
| Ala | Asn | Pro | Gln | Val | Glu | Gly | Gln | Leu | Gln | Trp | Leu | Ser | Gln | Arg |
| Ala | Asn | Ala | Leu | Leu | Ala | Asn | Gly | Met | Lys | Leu | Thr | Asp | Asn | Gln |
| Leu | Val | Val | Pro | Ala | Asp | Gly | Leu | Tyr | Leu | Ile | Tyr | Ser | Gln | Val |
| Leu | Phe | Ser | Gly | Gln | Gly | Cys | Arg | Ser | Tyr | Val | Leu | Leu | Thr | His |
| Thr | Val | Ser | Arg | Phe | Ala | Val | Ser | Tyr | Pro | Asn | Lys | Val | Asn | Leu |
| Leu | Ser | Ala | Ile | Lys | Ser | Pro | Cys | His | Arg | Glu | Thr | Pro | Glu | Glu |
| Ala | Glu | Pro | Met | Ala | Trp | Tyr | Glu | Pro | Ile | Tyr | Leu | Gly | Gly | Val |
| Phe | Gln | Leu | Glu | Lys | Gly | Asp | Arg | Leu | Ser | Thr | Glu | Val | Asn | Gln |
| Pro | Glu | Tyr | Leu | Asp | Leu | Ala | Glu | Ser | Gly | Gln | Val | Tyr | Phe | Gly |
| Ile | Ile | Ala | Leu | | | | | | | | | | | |

wherein Gln stands for a glutamine residue, Asp an aspartic acid residue, Pro a proline residue, Tyr a tyrosine residue, Val a valine residue, Lys a lysine residue, Glu a glutamic acid residue, Ala an alanine residue, Asn an asparagine residue, Leu a leucine residue, Phe a phenylalanine residue, Gly a glycine residue, His a histidine residue, Ser a serine residue, Thr a threonine residue, Ile an isoleucine residue, Trp a tryptophan residue, Arg an arginine residue, Met a methionine residue, and Cys a cysteine residue.

In further aspect of the present invention, there is provided a deoxyribonucleic acid comprising at least one base sequence selected from the group consisting of a base sequence represented by the following formula (II) and a complementary base sequence to said base sequence:

```
TCA GCT TCT CGG GCC CTG AGT GAC AAG CCT CTA GCC CAC GTA GTA
GCA AAC CCG CAA GTG GAG GGC CAG CTC CAG TGG CTG AGC CAG CGT
GCG AAC GCC CTG CTG GCC AAC GGC ATG AAG CTC ACG GAC AAC CAG
CTG GTG GTG CCG GCC GAC GGG CTG TAC CTC ATC TAC TCC CAG GTT
CTC TTC AGC GGT CAA GGC TGC CGC TCC TAC GTG CTC CTC ACT CAC
ACT GTC AGC CGC TTC GCC GTC TCC TAC CCG AAC AAG GTC AAC CTC
CTC TCT GCC ATC AAG AGC CCC TGC CAC CGG GAG ACC CCC GAG GAG
GCT GAG CCC ATG GCC TGG TAC GAG CCC ATC TAC CTG GGC GGC GTC
TTC CAG TTG GAG AAG GGT GAC CGG CTC AGC ACC GAG GTC AAC CAG
CCT GAG TAC CTG GAC CTT GCC GAG TCC GGG CAG GTC TAC TTT GGG
ATC ATT GCC CTG
```

wherein A stands for a deoxyadenylic acid residue, G a deoxyguanylic acid residue, C a deoxycytidylic acid residue and T thymidylic acid residue and wherein the left end and right end of the formula (II) represent 5'-hydroxyl group side and 3'-hydroxyl group side, respectively.

The DNA of the present invention includes a DNA comprising a base sequence having ATG (A, T and G are as mentioned above) attached to the 5'-end of the above-mentioned base sequence in order to produce mature TNF by means of culture of a microorganism or cell. The DNA of the present invention also includes a DNA having a partial or entire 5'-flanking DNA having a base sequence represented by the following formula (III):

4

```
ATGAGCACTGAGAGTATGATCCGGGACGTCGAGCTGGCGGAGGGGCCGCTCCCCAAGAAGG
CAGGGGGGGCCCCAGGGCTCCAAGCGTTGCCTCTGCCTCAGCCTCTTCTCTTTCCTGCTCGT
GGCTGGAGCCACCACGCTCTTCTGCCTGCTGCACTTCAGGGTGATCGGCCCTCAGGAGGAA
GAGCAGTCCCCAAACAACCTCCATCTAGTCAACCCTGTGGCCCAGATGGTCACCCTCAGA
```

The structure of a DNA and the structure of the polypeptide deduced therefrom may be partially changed by natural or artificial mutation without causing the main activity of the polypeptide to be changed. Hence, the DNA of the present invention may alternatively have a base sequence that codes for a polypeptide with a structure corresponding to that of a homologous variant of any of the aforementioned polypeptides.

In accordance with degeneracy of genetic code, it is possible to substitute at least one base of the base sequence of a gene by other kind of base without causing the amino acid sequence of the polypeptide produced from the gene to be changed. Hence, the DNA of the present invention may also have any base sequence that has been changed by substitution in accordance with degeneracy of genetic code. In this instance, the amino acid sequence deduced from the base sequence obtained by the above-mentioned substitution is identical with the amino acid seqeunce of the formula (I) as defined before.

In still a further aspect of the present invention, there is provided a replicable recombinant DNA which comprises the above-mentioned deoxyribonucleic acid according to the present invention and a replicable expression vehicle. The recombinant DNA is capable, in a transformant microorganism or cell culture, of expressing a polypeptide comprising the amino acid sequence of TNF. As the suitable vehicle, there may be mentioned, for example, pTNF-lacUV5-1 expression vehicle.

Further, the present invention is directed to a microorganism or cell culture transformed with a recombinant DNA capable of expressing a polypeptide comprising the amino acid sequence of TNF. Examples of such microorganism or cell culture include *Escherichia coli, Bacillus subtilis*, yeasts and higher animal cells.

In an even further aspect of the present invention, there is provided a method for producing the physiologically active polypeptide of the present invention which comprises:

(a) ligating the deoxyribonucleic acid of the formula (II) as defined above to a replicable expression vehicle to obtain a replicable recombinant DNA comprising said deoxyribonucleic acid and said replicable expression vehicle;

(b) transforming cells of a microorganism or cell culture with said replicable recombinant DNA to form transformants;

(c) selecting said transformants from parent cells of the microorganism or cell culture;

(d) incubating said transformants, causing said transformants to express said deoxyribonucleic acid and produce a physiologically active polypeptide; and

(e) isolating said physiologically active polypeptide from the incubated transformants.

According to the method of the present invention, the above-described polydeoxyribonucleic acid of the present invention is ligated to a replicable expression vehicle as a vector to obtain a replicable recombinant DNA containing the above-mentioned polydeoxyribonucleic acid. A microorganism or cell culture is transformed with the thus obtained replicable recombinant DNA to obtain a transformant microorganism or cell culture containing the recombinant DNA. The thus obtained transformant is isolated from the parent microorganism or cell culture by means of a phenotypical trait imparted with the DNA. The thus obtained transformant microorganism or cell culture is grown to effect expression of the genetic information that is encoded on the above-mentioned deoxyribonucleic acid, thereby producing a physiologically active polypeptide according to the present invention.

Thus, according to the process of the present invention, the physiologically active substance is obtained in substantially pure form.

Furthermore, the present invention is directed to a TNF, in mature form, secreted from host cells as a direct expression product. As a process for obtaining such a mature TNF, there may be mentioned, for example, a process comprising constructing a DNA sequence so as to bond an amino acid sequence, known as a signal peptide, composed of from 15 to 40 amino acids that is derived from a microorganism or higher animal to the N-terminus of the amino acid sequence of the mature TNF.

Now, an illustrative explanation will be given as to how the amino acid sequence of the present polypeptide and the base sequence of a DNA coding for the present polypeptide have successfully been determined.

1. Rabbits are injected with a substance having a capacity for stimulating the reticuloendothelial system.

2. The rabbits are then bred for 5 to 15 days and treated with endotoxin derived from Gram-negative bacteria to obtain the serum thereof. The serum exhibits cytotoxic activity against L cells and antitumor activity. The substance which exhibits such actvities is hereinafter referred to as "serum TNF."

3. Rabbits injected with the substance having a capacity for stimulating the reticuloendothelial system are bred for 5 to 15 days and then subjected to tracheotomy to obtain cells from alveolar washings.

4. The obtained cells from the alveolar washings are cultured in the presence of endotoxin derived from Gram-negative bacteria.

5. The supernatant of the cell culture exhibits cytotoxic activity against L cells. Such activity is eliminated by an antiserum (hereinafter referred to as "anti-TNF antibody") obtained from a mouse treated with the serum TNF. The supernatant of the cell culture to which endotoxin has not been added exhibits no cytotoxic activity against L cells.

6. Radioactive methionine is further added to the cell culture to which endotoxin has been added. The supernatant of the cell culture is analyzed by SDS-polyacrylamide gel electrophoresis. The analysis shows that a protein having a molecular weight of $17500\pm2000$ is formed. On the other hand, in the supernatant of the cell culture to which endotoxin has not been added, the formation of such protein is not observed.

7. The above-mentioned supernatant of the cell culture to which endotoxin has been added is subjected to SDS-polyacrylamide gel electrophoresis for fractionation. As a result, the fraction at the position corresponding to the molecular weight of $17500\pm2000$ is found to have a cytotoxic activity against L cells. The result of the analysis of rabbit serum TNF by the SDS-polyacrylamide gel electrophoresis is in agreement with the above result.

8. Cells are collected from the above cell culture and cytoplasmic ribonucleic acid ("ribonucleic acid" is hereinafter referred to as "RNA") is extracted in the presence of ribonuclease inhibitor.

9. From the cytoplasmic RNA, fractions adsorbed onto an olgio(dT)-cellulose column [hereinafter referred to as "mRNA" (messenger RNA)] is obtained.

10. The above-obtained mRNA is fractionated by sucrose gradient centrifugation.

11. The above fractions are separately injected into oocytes of Xenopus laevis and the cells are cultured in media. The media and the oocytes are evaluated for cytotoxic activity against L cells and the fractions exhibiting cototoxic activity against L cells are collected.

12. The cytotoxic activity against L cells which the above oocty es of Xenopus leavis exhibit is eliminated by the anti-TNF antibody.

13. The fractionated mRNA is converted into a single stranded DNA complimentary to the mRNA. Then, a double stranded DNA is prepared from the single stranded DNA. An oligomer of deoxycytidylic acid is added to the 3'-end of the double stranded DNA and inserted into a vector such as plasmid having one or more phenotypic markers.

14. With the so prepared recombinant, cell of *E. coli* are transformed, whereby transformants containing the double stranded DNA having a base sequence complementary to the base sequence of the mRNA are obtained.

15. Now referring back to the stage 2 mentioned above, in the stage 2 there is obtained a serum TNF in partially purified form. The serum TNF is subjected to SDS-polyacrylamide gel electrophoresis for further purification. Then the amino acid sequence of the thus highly purified TNF is determined.

16. Oligodeoxyribonucleotides are synthesized based upon the amino acid sequence of the highly purified serum TNF, and the so obtained oligodeoxyribonucleotides are labelled using a radioactive material.

17. The transformants prepared in the stage 14 mentioned above are transferred on a filter and the DNA of each of the thus transferred transformants is hybridized with the labelled oligo DNA obtained in the stage 15 mentioned above. The DNA having the desired genetic material incorporates the labelled oligo DNA and is identified by its radioactivity. Hybridizing colonies are selected.

18. A plasmid is isolated from each of the colonies selected in the stage 17 mentioned above, and each plasmid is cleaved with restriction enzymes and analyzed. Strains containing plasmids having same DNA fragments are selected from the colonies.

19. Plasmids are isolated from the above-obtained strains, and the base sequence of the DNA fragment inserted in the stage 13 mentioned above of each plasmid is determined. It is confirmed that the amino acid sequences deduced from the thus determined base sequence is in agreement with the amino acid sequence of the TNF determined in the stage 15 mentioned above. Thus, the base sequence of the DNA coding for a physiologically active polypeptide and the amino acid sequence of the physiologically active polypeptide are determined.

20. The DNA coding for the TNF is inserted into the downstream of the promoter of an appropriate expression vehicle to obtain a recombinant DNA. With the thus obtained recombinant DNA is transformed an appropriate host cell. The transformed host cell is then cultivated on a culture medium, thereby allowing the host cell of express the desired TNF.

21. The TNF activity of the thus obtained TNF is eliminated by the anti TNF antibody.

The foregoing discloses the procedures for obtaining the TNF gene and the base sequence of the DNA coding for TNF. However, it should be understood that the foregoing disclosure is not intended to limit the invention and that obvious changes may be made by those skilled in the art without changing the essential characteristics and the basic concept of the invention.

As mentioned above, cytoplasmic RNA containing mRNA coding for TNF can be obtained from cells from alveolar washings treated with endotoxin. Further, such cytoplasmic RNA may also be obtained by treating with endotoxin rabbit cells other than cells from alveolar washings, for example, cells adherent to plastics found in peripheral blood or endotoxin sensitive cells derived from peripheral blood, liver, spleen and the like.

In addition, mRNAs may also be obtained by connecting chromosome DNA to an appropriate vector, followed by insertion into a mammalian cell. For example, there is well known a recombinant method in which SV40 is used as the vector and COS as the host cell [see, for example, Mantei et al, Nature, vol. 281, p. 40 (1979); and Mellon et al, Cell, Vol. 27, p. 279 (1981)].

The thus obtained mRNAs are converted into cDNAs. A DNA is selected from the cDNAs by the hybridization method using a probe. The DNA is utilized for the production of the desired polypeptide by recombinant DNA technique.

Due to the variable use frequency of a codon (genetic code) corresponding to each amino acid and for other reasons, a partial or entire portion of the base sequence of the DNA coding for TNF may be substituted by an organochemically synthesized artificial DNA without causing the amino acid sequence of the polypeptide obtained therefrom to be changed.

Presumably, the TNF may be intracellularly produced in immature form as a prepeptide or prepropeptide, which may be grown via an intermediate form to a mature TNF in the processing stage. The immature form of TNF may be deduced from the base sequence of the TNF gene. The TNF DNA comprising a DNA encoding the TNF in immature or intermediate form may also be recombined with a natural or artificially synthesized DNA.

One application of this technique may be attained by inserting the methionine codon (ATG) in the 5'-end and inserting at least one stop codon selected from TAA, TAG and TGA in the 3'-end of the mature or intermediate of immature TNF DNA. Due to the presence of the methionine codon, the mature or intermediate or immature TNF may be produced on the mRNA synthesized with the aid of an appropriate promoter. However, the methionine residue attached to the N-terminus of the TNF is cleaved or not cleaved according to the kind of the host cell employed. The purpose of inserting the stop codon is to stop translation of the mRNA transcribed from the TNF DNA at an appropriate position (C-terminus of polypeptide of the formula I).

Another application of this technique may be attained by adding to the DNA a base sequence coding for a highly hydrophobic peptide having an amino acid sequence known as a "signal sequence". By this addition, it may become feasible to secrete the TNF to outside the host cell or, in the case of Gram-negative bacteria, into the space known as "periplasm".

When a vector in which a start codon is incorporated is employed, a fused peptide may be produced which consists of the TNF and a peptide attributed to the vector. In this case, the fused peptide may be cleaved chemically or enzymatically. Alternatively, the fused peptide, if the main activity of the TNF is not adversely affected, may be used as it is.

The TNF DNA may be connected, at its region upstream of the 5'-end, to the gene sequence of a promoter thereby to obtain a TNF DNA-promoter sequence which does not hinder its replication and does not cause translation of the resultant RNA to be adversely affected. The thus obtained TNF DNA-promoter sequence may be combined with a vector which is replicable in a bacterium or higher organism cell to obtain a recombinant gene. The thus obtained recombinant gene may be used to transform a bacterium or higher organism cell used as a host. The thus obtained transformant may be cultured to effect expression of the TNF gene in order to produce the TNF.

When *Escherichia coli* is used as the above-mentioned host, there may be mentioned, as the suitable host, various mutant strains of *E. coli* K-12, such as HB101(ATCC33694), C600K(ATCC33955), D1210, RRI(ATCC31343), MC1061, LE392 (ATCC33572), JM101(ATCC33876), JM103(Ray Wu et al, Method in Enzymology, Vol. 101, Academic Press, New York) and χ1776 (ATCC31244). When the *E. coli* host is employed, there may be mentioned, as the suitable vector, plasmids such as pBR322, pBR325, pBR327, pUC8, pUC9, pMB9 (ATCC37019), pJB8(ATCC37074) and pKC7(ATCC37084), λ phages such as λgt, λB and Charon 4A and M13 phage. To have TNF produced in the *E. coli* cell, a promoter selected from the promoters of the *E. coli* and phage genes may be employed. Examples of the suitable promoter include the genes for lactose degradation enzyme (LAC), UV5 mutant thereof, penicillinase (BLA) and tryptophan synthetase (TRP), λ phage $P_L$ promoter and TAC promoter which is a fused promoter of tryptophan synthetase and lactose degradation enzyme.

When *Bacillus subtilis* is used as the host, there may be mentioned, as the suitable host, BD170 strain (ATCC33608), BR151 strain (ATCC33677) and MI112 strain (ATCC33712). When the *Bacillus subtilis* host is employed, there may be mentioned, as the suitable vector, plasmids, pC194(ATCC37034), pUB110(ATCC37015), pSA2100(ATCC37014) and pE194. Further, when the *Bacillus subtilis* host is employed, there may be mentioned, as the suitable promoter, the genes for chloramphenicol acetylation enzyme (CAT), penicillinase and anti-erythromycin.

When a yeast is used as the host, there may be mentioned, as the suitable host, strains of *Saccharomyces cerevisiae* such as RH218(ATCC44076), SHY1(ATCC44769), SHY3(ATCC44771), D131A, 483 and 830. When the yeast host is employed, there may be mentioned, as the suitable vector, plasmids such as YEp13(ATCC37115), YEp6, YRp7 and YIp5. Further, when the yeast host is employed, there may be mentioned, as the suitable promoter, the genes for acid phosphotase, alcohol dehydrogenase (ADHI), tryptophan synthetase (TRP), phosphoglycerate kinase (PGK), cytochrome B(COB) and actin.

When a higher organism cell culture is used as the host, there may be mentioned, as the suitable host, the cell cultures of monkey kidney, COS and mouse C127(ATCC1616). When the higher organism cell culture host is employed, there may be mentioned, as the suitable vector, SV40 and bovine polyomavirus.

The novel physiologically active polypeptide of the present invention induces necrosis of tumors with no toxic effect upon the normal tissues of the living body. The active polypeptide of the present invention

# 0 148 311

may be formulated according to known methods to prepare pharmaceutical compositions which are useful for the inhibition of cell proliferation, e.g. malignant tumor cells proliferation. The active polypeptide may be combined in admixture with a pharmaceutically acceptable carrier vehicle. An effective amount of the active polypeptide of the present invention may be mixed with a suitable amount of vehicle in order to prepare pharmaceutically acceptable compositions suitable for effective administration to the recipient.

The physiologically active polypeptide of the present invention may be administered, to subjects requiring antitumor or antiviral treatment, as an injection, eye drop, nasal drop, inhalant, external preparation, oral administration, rectal administration or vaginal tablet. The daily dose of the polypeptide of the present invention per adult may be generally in the range of from 50 to 100,000,000 units. It may be preferably in the range of from 50 to 500,000 units in the case of local administration, from 1,000 to 1,000,000 units in the case of general injection such as intravenous injection and intramuscular injection, and from 10,000 to 100,000,000 units in the case of oral administration. The daily dose may be increased or decreased according to the direction for use and symptom of recipient.

The terminology "1 unit" used above means a quantity of the physiologically active polypeptide of the present invention by which 50% of $1 \times 10^5$ cells/ml of L—M cells (American Type Culture Collection CCL 1.2) are killed. The above-mentioned quantity is measured as follows. As culture vessels, there are employed 96-well microtiter plates produced by flow Laboratories, Inc. (U.S.A.), and L—M cells are cultured in Eagle's minimum essential medium containing 1 v/v% of bovine fetal serum [the composition of this medium is described, for example, in Tissue Culture, edited by Junnosuke Nakai et al, Asakura Shoten, Japan (1967)]. A sample (0.1 ml) serially diluted with the medium and the L—M cell suspension (0.1 ml, $1 \times 10^5$ cells/ml) are mixed into each well of the plates and the plates are incubated at 37°C for 48 hours in an air containing 5% carbon dioxide. At the end of the culture period, 20 μl of glutaraldehyde is added to fix the cells. After fixation, the plates are washed with distilled water and allowed to dry, and 0.05% methylene blue (0.1 ml) is added to stain the viable cells. The plates are thoroughly washed with distilled water to remove excess dye and allowed to dry. 0.36 N Hydrochloric acid is added to each well to extract the dye from stained cells. Absorbance of each well at 665 nm is measured with Titertek Multiskan produced by Flow Laboratories. Inc. The absorbance is proportional to the number of viable cells. The above-mentioned quantity of the physiologically active polypeptide of the present invention by which 50% of $1 \times 10^5$ cells/ml of L—M cells are killed is obtained by plotting the dilution versus the absorbance on a graph.

The physiologically active polypeptide of the present invention may be suitably administered parentially. In the parenteral preparation, there may be incorporated, as an additive, a stabilizer such as albumin, gelatin, globulin, protamine, salt of protamine or the like, a thickner such as sucrose, glycerine, methylcellulose, carboxymethylcellulose or the like and/or a pH adjusting agent such as various inorganic salt. The polypeptide may also be suitably administered in the form of a tablet. In the tablet, there may be incorporated, as an additive, a vehicle such as starch, lactose or the like in addition to the above-mentioned stabilizer.

Accordingly, in a further aspect of the present invention, there is provided a pharmaceutical composition comprising an effective antitumor or antiviral amount of a physiologically active polypeptide as defined herein and at least one pharmaceutically acceptable carrier, diluent or excipient.

As a result of animal experiment, it has been found that a mouse tumor is completely healed by one or two injections only, in most cases. In particular, an aliquot of artificial neoplastic cells (Meth-A cells) was transplanted to the skin of each mouse. When the tumor grew to have a diameter of 6 to 7 mm, as little as 0.6 μg of the polypeptide of the present invention was injected. A week later, a scab appeared. Two weeks later, hairs began to grow, which meant complete healing of the tumor. More later, pregnancy and successful birth were observed for the mice.

Accordingly, in a further aspect of the present invention, there is provided a method for treating tumors by administering to a host an effective antitumor amount of a physiologically active polypeptide as defined herein.

The present invention will be described in more detail with reference to the following Examples which should not be construed to be limiting the scope of the present invention.

In practicing the present invention, construction of a recombinant DNA and insertion of a recombinant DNA to a microorganism were carried out in accordance with the procedure described in the following experimental reports [Literatures (1) to (4)], unless otherwise indicated.

(1) Yasutaka Takagi, Manual for Genetic Engineering, Kodan-sha, Tokyo.

(2) Yasutaka Takagi, Experimental Method in Genetic Engineering, Kodan-sha, Tokyo.

(3) T. Maniatis, E. F. Fritsch, J. Sam Brook, Molecular Cloning, Cold Spring Harbor Laboratory, New York.

(4) Ray Wu et al., Method in Enzymology, Vol. 101, Academic Press, New York.

Abbreviations used in Examples

| | |
|---|---|
| LB medium: | Lauria-Bertani medium |
| DMSO: | dimethylsulfoxide |
| EDTA: | ethylenediaminetetraacetic acid |
| SDS: | sodium dodecyl sulfate |
| lac: | lactose |

| Tris: | tris(hydroxymethyl)aminomethane |
| XAR-5: | X-ray film manufactured and sold by Eastman Kodak Company, U.S.A. |
| 5×SSC: | 0.75 M NaCl+0.075 M sodium citrate, pH7 |
| PTH: | phenylthiohydantoin |
| IPTG: | isopropyl thiogalactoside |
| x-gal: | 5-dibromo-4-chloro-3-indolylgalactoside |
| TAE: | 0.04 M Tris-acetate (pH8.0)—0.002 M EDTA |
| 5×Denhardt solution: | an aqueous solution containing Ficoll 1000 mg, polyvinyl-pyrrolidone 1000 mg and BSA 1000 mg per liter |
| bp: | base pair |

Example 1
(Evaluation of cytotoxic activity against L cells)

The evaluation of the cytotoxic activity of the physiologically active substance prepared in the following Examples against L cells was effected by measuring its cytotoxic effect on the L929 cells (American Type Culture Collection CCL1), in accordance with the method of Ruff et al [see Lymphokines, Vol. 2, edited by E. Pick, Academic Press, N.Y., p 235 (1980)] or the method described in J. Immunol, *126*, p 235 (1981). The method of evaluation of the cytotoxic activity of the physiologically active substance prepared in the following Examples is explained below.

As culture vessels, there are employed 96-well microtiter plates produced by Flow Laboratories, Inc. (U.S.A.), and L929 cells are cultured in Eagle's minimum essential medium containing 1 v/v% fetal calf serum and 5 µg/ml (final concentration) of actinomycin D [the composition of this medium is described, for example, in Tissue Culture, edited by Junnosuke Nakai et al, Asakura Shoten, Japan (1967)]. A sample (0.1 ml) serially diluted with the medium and the L929 cell suspension (0.1 ml, $1 \times 10^5$ cells) are mixed in each well of the plates and the plates are incubated at 37°C for 21 hours in an air containing 5% carbon dioxide. At the end of the culture period, 20 µl of a 20% aqueous solution of glutaraldehyde is added to fix the cells. After fixation, the plates are washed with distilled water and allowed to dry, and 0.05% methylene blue (0.1 ml) is added to stain the viable cells. The plates are thoroughly washed with distilled water to remove excess dye and allowed to dry, 0.36 N Hydrochloric acid is added to each well to extract the dye from stained cells. Absorbance of each well at 665 nm is measured with Titertek Multiskan (produced by Flow Latoratories, Inc. U.S.A.). The absorbance is proportional to the number of viable cells. The cytotoxic activity of the physiologically active substance, unit/ml, is defined as the reciprocal dilution of the physiologically active substance that causes 50% cytotoxicity, and can be obtained by plotting the dilution versus the absorbance on a graph.

On the other hand, the amount of protein was determined by a method in which Coomassie Brilliant Blue G250 is bonded to protein, according to the teaching of Branford et al [see Anal. Biochem. Vol. 72, pp 248—254 (1976)].

Example 2
Step 1
(Preparation of TNF from rabbit serum)

Nihon-hakushokushu (Japanese White) female rabbits, weighting 2.5 to 3 kg, are injected with 50 mg of formalin-killed Propionibacterium acnes (Coryne-bacterium parvum; Wellcome Research Laboratories, England) through the ear vein. Eight days later, 100 µg of endotoxin (lipopolysaccharide from *Escherichia coli* 026:B6, produced by Difco Laboratories, U.S.A.) was injected again through the ear vein and 2 hours later whole blood was collected from the heart. To the collected blood, heparin sodium was added in an amount of 100 units per 100 ml. The blood was then centrifuged while cooling at 5,000 rpm for 30 minutes to remove blood cells and insoluble solids. As a result, a plasma (2.4 liters) having a serum TNF cytotoxic activity of $3 \times 10^4$ units/ml was obtained from 40 rabbits.

Step 2
(Partial purification of TNF from rabbit serum)

To the plasma (2.4 liters) obtained in Step 1, added was 24 g of cellite. The resultant was stirred for one hour, and then subjected to filtration. The filtrate was mixed with 1.2 liters of 0.04 M Tris-HCl buffer (pH 7.8), and then applied to a column of DEAE-Sepharose CL-6B (manufactured and sold by Pharmacia Fine Chemicals, Inc. Sweden) sufficiently equilibrated with 0.04 M Tris-HCl buffer (pH 7.8) containing 0.1 M NaCl. The column was washed with 0.04 M Tris-HCl buffer, and the adsorbed TNF was eluted with 0.04 M Tris-HCl buffer (pH 7.2) containing 0.18 M NaCl. Fractions exhibiting cytotoxic activities against L cells were concentrated by ultrafiltration. The so obtained concentrate was applied to a column of Sephacryl S-200 (manufactured and sold by Pharmacia Fine Chemicals, Inc. Sweden) sufficiently equilibrated with 5 mM phosphate buffer, (pH 7.4) containing 0.15 M NaCl and gel-filtered using the same buffer. The active fractions were concentrated by ultrafiltration, whereby a purified TNF having an activity of $3.5 \times 10^6$ units and a specific activity of $18 \times 10^6$ units/mg protein was obtained.

9

Step 3
(Anti-TNF antibody)

The rabbit serum TNF partially purified in Step 2 was mixed with complete Freund's adjuvant (1:1 by volume), and then injected subcutaneously at the back of a 12 week age BALB/C male mouse. The above operation was repeated 2 and 4 weeks after the initial injection. One week after the last injection, whole blood was collected. From the collected blood, a serum was obtained.

The so-obtained serum was added to the culture medium for evaluation of the cytotoxic activity of TNF against L cells in such an amount that it was diluted 500-fold in final concentration. The cytotoxic activity of the rabbit serum TNF against L cells was evaluated in the same manner as described in Example 1. It was found that the rabbit serum TNF exhibited no cytotoxicity aganst L cells. From the above result, it can be concluded that the mouse serum obtained in this step contained an antibody to the rabbit serum TNF (anti-TNF antibody).

Example 3
Step 1
(Preparation of TNF-producing cells)

Nihon-hakushokushu (Japanese White) female rabbits weighing 2.5 to 3.0 Kg were injected intravenously with formalin-killed cells of Propionibacterium acnes (Coryne bacterium parvum; Wellcome Research Laboratories, England). Seven days later, the rabbits were subjected to tracheotomy, and the lungs were washed with a physiological saline solution, whereby floating cells were obtained. The so obtained cells were washed with a physiological saline solution. Using as a culture medium RPMI 1640 (Flow Laboratories Inc., U.S.A.) containing 10 v/v% fetal calf serum (Flow laboratories Inc. U.S.A.), the cells were incubated at 37°C in air containing 5% carbon dioxide. The cell culture was divided into two groups, and to one of them endotoxin derived from Escherchia coli (lipopolysaccharide from Escherchia coli 026:B6, produced by Difco Laboratories, U.S.A.) was added at a concentration of 10 µg/ml. The same amount of sterile water was added to the other. The supernatant of the cell culture to which endotoxin was added exhibited cytotoxic activity against L cells, and the activity reached the maximum value within seven hours after. Such activity was eliminated by the anti-TNF antibody, but was not eliminated by the normal mouse serum.

On the other hand, the supernatant of the cell culture to which no endotoxin was added exhibited no cytotoxicity against L cells.

Step 2
(Molecular weight of TNF)

To the cell culture prepared in Step 1 to which endotoxin was added, radioactive L-[$^{35}$S] methionine (1300 Ci/mmol, produced by Amersham International plc. England) was further added (1 mCi/ml). In accordance with the method of Laemmli [see Laemmli, U.K. (1970), Nature (London), Vol. 227, pp 680—685), the supernatant was analyzed by the SDS-polyacrylamide gel electrophoresis. The gel concentration was adjusted to 12.5 wt%. After the electrophoresis, the gel was treated with Enhance® (trade mark of a product of New England Neclear Inc., U.S.A.), and after drying, attached was X-ray film (Fuji RX, manufactured and sold by Fuji Photo Film Co., Ltd., Japan), followed by exposure to radiation. In the supernatant of the cell culture cultivated in the presence of endotoxin, it was observed that a substance having a molecular weight of about 17500 was formed.

Further, the supernatant of each cell culture prepared in Step 1 was subjected to SDS-polyacrylamide gel electrophoresis in the same manner as described above. Thereafter, the gel was shaken in 2.5% NP 40® (trade mark of a surface active agent manufactured and sold by Calbiochem, U.S.A.) for one hour, and then in water for two hours. After shaking, each migration lane was separated by cutting, and cut into strips of 2 mm-width in a direction perpendicular to the direction of migration. Each strip was cultured with L cells, and evaluated for cytotoxic activity against L cells. In the lane on which the supernatant of the cell culture containing endotoxin was developed, cytotoxicity against L cells was observed at a position corresponding to the molecular weight of about 17500. No cytotoxicity was observed at other positions.

Step 3
(Construction of mRNA)

The cell culture as prepared in Step 1 was incubated for 2 hours after addition of endotoxin, followed by centrifugation to collect cells. Extraction of cytoplasmic RNA from the collected cells and extraction of mRNA from the cytoplasmic RNA were effected in accordance with the method of Chirgwin et al [see Chirgwin, J. M. et al. Biochemistry, Vol. 18, p 294 (1979)]. 4 ml of a 4 M guanidine thiocyanate solution was added to $3 \times 10^8$ cells, and the mixture was pulverized by means of a homogenizer (Model: AM-7, manufactured and sold by Nihon Seiki Seisakusho, Japan). The residues were removed by centrifugation, and 2.4 g of cesium chloride was dissolved therein. The mixture was carefully poured into a polyallomer tube in which 2.5 ml of 5.7 M cesium chloride and 0.1 M EDTA solution (pH 7.5) had been loaded in advance, and then subjected to ultracentrifugation at 30,000 rpm for 12 hours at 20°C using Beckman SW41 Ti rotor (manufactured and sold by Beckman Instrument, U.S.A.). After removal of the supernatant, the pellet was dissolved in 1 ml of 10 mM Tris-HCl buffer (containing 5 mM EDTA and 1 w/v% SDS). The resulting solution was extracted with a 4:1 by volume mixture of chloroform and 1-butanol. To the aqueous phase, 0.05 volume of 2 M sodium acetate and 2.5 volumes of ethanol were added, and allowed to stand at −20°C for 2 hours or more, thereby to precipitate RNA. The precipitate was collected by centrifugation, dried, and then dissolved in 500 μl of sterile water. As a result, a cytoplasmic RNA solution was obtained.

The above-obtained RNA solution was heated at 68°C for 2 minutes, and thereafter, chilled quickly, 500 μl of 2-fold concentration 10 mM Tris-EDTA buffer (pH 7.4) (containing 1 mM EDTA, 0.1 w/v% SDS and 0.5 M lithium chlorate) was added to the solution, and the mixture was applied to a 200 mg oligo (dT)-cellulose (manufactured and sold by Bethesda Research Laboratories, Inc., U.S.A.) column, and washed with 10 ml of the same buffer (one-fold concentration) as described above. The material retained by the column was eluted with 2 ml of an elution buffer containing 10 mM Tris-HCl buffer pH 7.4, 1 mM EDTA and 0.1 w/v% SDS. To the eluate, added was 0.05 volume of sodium acetate and 2.5 volumes of ethanol, and the mixture was cooled at −20°C to precipitate. The precipitate was collected by centrifugation, and applied to the oligo (dT)-cellulose column, and the fractions adsorbed onto the oligo (dT)-cellulose were collected. 85 μg of mRNA was recovered as determined by the ultraviolet spectrum analysis.

Step 4
(Size fractionation of mRNA)

880 μg of mRNA prepared by the same method as described in Step 3 was dissolved in 250 μl of water, and the resulting solution was superposed on 10 ml of a 5—25% linear sucrose density gradient. The sucrose density gradient was prepared by means of ISCO 570 gradienter (manufactured and sold by ISCO Inc., U.S.A.), using Tris buffer solutions (containing 25 mM Tris-HCl (pH 7.2), 2 mM EDTA and 1 w/v% SDS] respectively containing 5% sucrose and 25% sucrose.

Using Beckman SW41 Ti rotor, ultracentrifugation was effected at 40000 rpm for 12 hours at 4°C, and fractions each of 400 μl were recovered by means of a fraction recovering apparatus (manufactured and sold by Beckman Instrument, U.S.A.), and then ethanol precipitated. The precipitated fractions were centrifuged, and dissolved in sterile water.

Step 5
(Experiment on translation of mRNA)

Translation of mRNA using oocytes of Xenopus laevis (Hamamatsu biological teaching materials) was conducted according to the procedure described in the experimental reports (for example, Hiroshi Teraoka, Mikio Itsuki and Kentaro Tanaka, "Protein, Nucleic acid, Enzyme", Genetic Engineering, extra edition., 1981, p 602). Xenopus laevis was procured from Hamamatsu biological teaching materials. Fractionated mRNA obtained in Step 4 was dissolved in sterile water to have a concentration of 1 μg/μl, and the solution was injected into oocytes in such a small amount as 50 nl per cell. Cells were then cultured for 24 hours in a Barth solution [containing 7.5 mM Tris-HCl (pH 7.6), 88 mM NaCl, 1 mM potassium chloride, 0.33 mM calcium nitrate, 0.41 mM calcium chloride, 0.82 mM magnesium sulfate, 2.4 mM sodium bicarbonate, 18 U/ml penicillin G and 18 μg/ml streptomycin] which contains 1 mg/ml bovine serium albumin. Oocytes were crushed, in the culture liquid, by means of a glass bar. The culture liquid was then centrifuged, and the supernatant was evaluated for the cytotoxic activity against L cells. mRNA which would be translated to give a polypeptide having maximum activity sedimented as 16 S in size. This activity was dissipated by the anti-TNF antibody obtained in Step 3 of Example 2, but was not dissipated by the normal mouse serum.

Step 6
(Preparation of transformants)

Using 5 μg of the fractionated mRNA obtained in Step 4, a double stranded DNA was prepared in accordance with procedure described in Literature (1), from page 96. As the reverse transcriptase, use was made of a product of Life Science, Inc., U.S.A. The double stranded DNA was size-fractionated by electrophoresis on a 3.5% polyacrylamide gel, and 330 ng fraction of about 1000 to 2000 bp was obtained. In accordance with the procedure described in Literature (1), 7 ng of this fraction was extended with deoxyC residues using terminal deoxynucleotidyl transferase (manufactured and sold by Bethesda Research Laboratories, Inc., U.S.A.) and annealed with 56 ng of plasmid pBR322 which had been digested with PstI and extended with deoxyG residues. The so-annealed mixture was inserted into *E. coli* K-12 strain (HB101, ATCC33694) to transform the strain. As a result, 12000 transformants were obtained.

Step 7
(Partial amino acid sequence of rabbit TNF)

Rabbit TNF partially purified in Example 2 (activity: $5 \times 10^7$ units) was subjected to SDS-polyacrylamide gel electrophoresis for purification as in Step 2. Part of the gel was dyed with Coomassie Brilliant Blue. A band at the position corresponding to the molecular weight of about 17000 was cut out from the gel, and extracted with 1% ammonium bicarbonate. About 180 μg of TNF was recovered as protein.

150 μg of the recovered TNF was dissolved in 75 μg of 1% ammonium bicarbonate, followed by addition of 3 μg of TPCK trypsin (manufactured and sold by Worthington Biochemical, U.S.A.). The mixture was incubated at 37°C for 4 hours. The mixture was then fractionated by means of a high-speed liquid chromatography column comprising Cosmosil 5C8 (manufactured and sold by Nakarai Chemical, Ltd.

Japan) as the packing material, thereby to obtain fragments digested with trypsin.

The highly purified TNF and the trypsin-digested fragments thereof were then subjected to desalting by means of a column of Sephadex G-25 (manufactured and sold by Pharmacia Fine Chemicals, Inc., Sweden), and then freeze-dried. According to the method of R. M. Hewick et al (see J. Biol. Chem. Vol. 256. pp 7990—7997, 1981), the purified TNF and the trypsin-digested fragments were each subjected to Edman Degradation from the N-terminal. PTH-amino acid liberated in each step was analyzed by the customary method by means of a high-speed chromatography model SP8100 (manufactured and sold by Spectra physics, U.S.A.) using Solpacks ODS (manufactured and sold by E. I. Du Pont, U.S.A.) as the column. As a result, it was found that the TNF had the following N-terminal amino acid sequence: Ser-Ala-Ser-Arg-Ala-Leu-Ser-Asp-Lys-Pro-Leu-Ala-His-Val-Val-Ala-Asn-Pro-Gln-Val-Glu-Gly-Gln-Leu-Gln-One of the trypsin-digested fragments had the following N-terminal amino acid sequence.

Glu Thr Pro Glu Glu Ala Glu Pro Met Ala

Step 8
(Synthesis of oligodeoxynulcleotide probe)

Oligodeoxynucleotides complementary to the base sequence of the mRNA which was estimated from the amino acid sequence of rabbit TNF obtained in Step 7 of Example 3 was synthesized according to the improved phosphotriester method which had already been reported by the present inventor in H. Ito et al, "Nucleic Acid Res." 10, 1755—1769 (1982). In preparing oligodeoxynucleotides, 128 oligodeoxynucleotides estimated from the amino acid sequence of rabbit TNF were classified into five groups, namely groups of 16, 16, 32, 32 and 32 and were synthesized as mixtures of oligodeoxynuclotides of the respective groups. The obtained oligodeoxynucleotides of the respective groups were deprotected according to the customary method and purified by column chromatography using Sephadex G-50 (manufactured and sold by Pharmacia Fine Chemicals, Inc., Sweden), electrophoresis on a 20% by weight polyacrylamide gel containing 7M of urea and column chromatography using DE52 (manufactured and sold by Whatman Ltd., U.S.A.). The thus obtained oligodeoxynucleotides of the respective groups were dialyzed against 0.1 mM Tris-EDTA buffer solution.

Each of the purified oligodeoxynucleotides of the respective groups was labelled using $T_4$ polynucleotide kinase (manufactured and sold by Bethesda Research Laboratories, Inc., U.S.A.) and $\gamma$-$^{32}$P-adenosine triphosphate according to the customary method and then purified by column chromatography using DE52 (manufactured and sold by Whatman Ltd., U.S.A.). The radioactive material was incorporated into each of oligodeoxynucleotides of the respective groups in the amount of about $3\times10^8$ cpm/µg. The oligodeoxynucleotide probes each obtained in the form of a mixture of the respective group were designated as shown in Table 1.

Part of the amino acid sequence of the rabbit TNF, the base sequence of the mRNA estimated from the amino acid sequence of the rabbit TNF and the base sequences of synthetic oligodeoxynucleotide probes of the respective groups are shown in Table 1.

TABLE 1

| Amino acid sequence | Carboxyl terminal | Ala | Met | Pro | Glu | Ala | Glu | Glu | Amino terminal |
|---|---|---|---|---|---|---|---|---|---|
| mRNA | 3' | XCG | GTA | XCC | YAG | XCG | YAG | YAG | 5' |
| Probe MH | 5' | GC | CAT | MGG | MTC | GGC | MTC | MTC | 3' |
| Probe MI | 5' | GC | CAT | NGG | MTC | GGC | MTC | MTC | 3' |
| Probe MJ | 5' | GC | CAT | ZGG | MTC | AGC | MTC | MTC | 3' |
| Probe MK | 5' | GC | CAT | ZGG | MTC | CGC | MTC | MTC | 3' |
| Probe ML | 5' | GC | CAT | ZGG | MTC | TGC | MTC | MTC | 3' |

Note:

X represents a ribonucleic acid residue of A, C, G or U.
Y represents a ribonucleic acid residue of A or G.
M represents a deoxyribonucleic acid residue of T or C.
N represents a deoxyribonucleic acid residue of A or G.
Z represents a deoxyribonucleic acid residue of A, C, G or T.
The mRNA of the cells producing TNF which was obtained according to Step 3 of Example 3 was

12

treated with a solution containing 1 M of glyoxal, 10 mM of $NaH_2PO_4$ and 50% by volume dimethyl sulfoxide at 50°C for 60 minutes and then subjected to fractionation using electrophoresis on a 1.1% by weight agarose gel. The fractionated mRNA was transferred on a filter of an electrophoresis type transfer blotting apparatus (manufactured and sold by Bio Rad, U.S.A.) according to the manual of the maker. Then the mRNA on the filter of the apparatus was treated with a 5×Denhart solution containing a 5×SSC solution and 150 µg/ml of denatured salmon spermatozoon at 65°C for two hours and, then treated with a 5×Denhart solution containing $1\times10^7$ cpm/ml of the labelled oligodeoxynucleotides and a 5×SSC solution at 50°C for two hours. The above-obtained filter was washed with a 6×SSC solution successively four times at room temperature, 40°C, 50°C and 60°C. An XAR-5 X-ray film (manufactured and sold by Eastman Kodak Company, U.S.A.) was exposed to the radiation from the filter. As a result, it was found that the oligodeoxynucleotides designated by Probe MJ were most strongly hybridized with the mRNA, showing that the oligodeoxynucleotide having a base sequence which was completely complementary to the mRNA was contained in the oligodeoxyribonucleotides designated by Probe MJ.

Step 9
(Cloning of TNF gene of rabbit)
In accordance with the procedure described in Literature (2), page 162, the transformants obtained in Step 6 of Example 3 were transferred onto a cellulose filter and the DNA of the transformants was hybridized with the labelled oligodeoxynucleotide (Probe MJ) selected in Step 8 of Example 3 under the same conditions as in Step 8 of Example 3 (colony hybridization). In the just above procedure, 49 colonies which were strongly hybridized with the labelled oligodeoxynucleotides (Probe MJ) were selected and further fixed onto another nitrocellulose filter. Then, using 49 colonies, further hybridization was carried out to select nine colonies which were more strongly hybridized with the labelled oligodeoxynucleotides (Probe MJ).

In accordance with the rapid plasmid separating procedure described in Literature (1), page 6, about 5 µg plasmid was obtained from each of the nine colonies. Each of the obtained plasmids was cleaved using restriction enzymes, PstI, TaqI, RsaI and PvuII (each manufactured and sold by Bethesda Research Laboratories, Inc., U.S.A.) according to the procedure described in the manual of the maker, followed by electrophoresis effected on a 1% by weight agarose gel. Then, fragments obtained by cleavage by the respective restriction enzymes were compared with respect to length thereof.

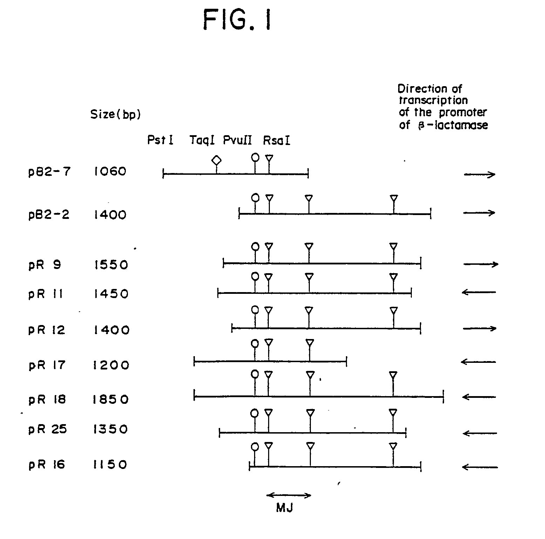

The results suggested that all the nine strains corresponding to the nine colonies had the base sequence of the fragment obtained by cleavage by PvuII and RsaI and consisting of about 50 bp and that eight of the nine strains had the base sequence of the fragment obtained by cleavage by RsaI and consisting of about 200 bp. In other words, the results suggested that the nine strains had the partially common base sequences. The results of analysis by the restriction enzymes are shown in Fig. 1.

Seven strains containing plasmids designated in Table 2 below were separately cultivated in 2 ml of LB medium containing 10 µg/ml of tetracycline so that the optical density of the solutions showed the values shown in Table 2 below, followed by centrifugation to obtain respective strains. The obtained strains are separately added into 2 ml of physiological saline and disrupted by sonication. The obtained solutions are subjected to centrifugation and the cytotoxic activity against L cells of the obtained supernatants was determined. The results are shown in Table 2 below. As a blank test, the same procedures as mentioned above were repeated using a strain containing plasmid pBR322. The results are also shown in Table 2 below.

# 0 148 311

TABLE 2

| Plasmid | Number of annealed base pairs | $OD_{600}$ | Cytotoxic activity against L cells (unit/ml) |
|---|---|---|---|
| pB2—2 | 1400 | 1.369 | 35 |
| pB2—3 | 800 | 1.605 | <10 |
| pB2—7 | 1060 | 1.364 | <10 |
| pR9 | 1550 | 1.618 | <10 |
| pR12 | 1400 | 1.458 | 15 |
| pR18 | 1850 | 1.438 | <10 |
| pR25 | 1350 | 1.514 | <10 |
| pBR322 | 0 | 1.677 | <10 |

The cytotoxic activity against L cells was eliminated by anti-TNF antibody but was not eliminated by normal mouse serum. This shows that all of the above-mentioned nine colonies have plasmids which contain oligodeoxynucleotides coding for TNF.

Step 10
(Determination of base sequence of DNA coding for rabbit TNF)

E. coli strains containing plasmids pB2—7 and pR18 were cultivated in one liter of M9 medium described in Literature (3), page 440 and containing 10 µg/ml of tetracycline. Then, in accordance with procedure described in Literature (3), page 90, each of plasmids was isolated in an amount of about 150 µg.

The base sequence of each plasmid was determined according to the Maxam-Gilbert chemical procedure described in Maxam et al "Method in Enzymology", 55, P490 (1980), Academic Press. The thus determined base sequence was found to be in agreement with the partial amino acid sequences determined in Step 7 of Example 3. Thus, the whole sequence of TNF of rabbit was considered to be elucidated.

Step 11

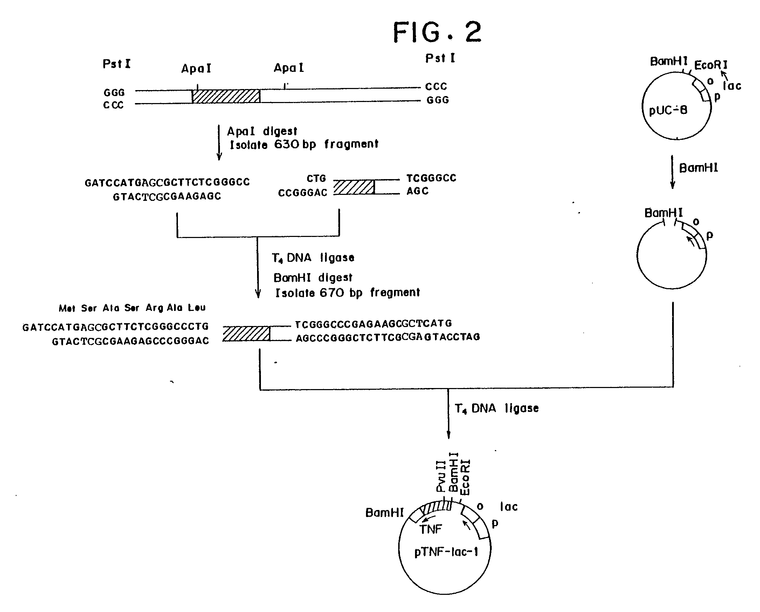

In this step, construction of a plasmid was carried out using the recombinant plasmid pR12 to obtain direct expression of TNF in E. coli using lac as a promoter. The procedures are illustratively shown in Fig. 2. First 10 µg of plasmid pR12 was digested with 10 units of Apal (manufactured and sold by Bethesda Research Laboratories, Inc., U.S.A.) at 37°C for two hours and electrophoresed on a 4% by weight polyacrylamide gel to isolate about 630 bp fragments. About 1 µg of the fragment was isolated from the gel by electroelution. In the same manner as in Step 8 of Example 3, two oligodeoxynucleotides shown in Fig. 2, namely 5'-GATCCATGAGCGCTTCTCGGGCC-3' and 5'-CGAGAAGCGCTCATG-3' were synthesized. Then, each 5' end of the oligodeoxynucleotides (about 100 pmole) was phosphorylated using $T_4$ polynucleotide kinase in accordance with the method described in Literature (3), page 122. After completion of the reaction, the reaction mixture was extracted with phenol and then with chloroform. Then the obtained synthetic oligomers were mixed with 0.5 µg of the Apal 630 bp fragment and ethanol precipitated. The fragment was ligated with the synthetic oligomers at 4°C overnight using 10 units of $T_4$ DNA ligase in accordance with the procedure described in Literature (1), page 37. After completion of the reaction, the reaction mixture was ethanol precipitated and digested with 20 units of BamHI at 37°C for three hours, followed by electrophoresis effected on a 4% by weight polyacrylamide gel to recover about 670 bp fragment by electroelution. One µg of commercially available plasmid pUC-8 (catalog No. 4916, manufactured and sold by P—L Biochemicals, Inc., U.S.A.) was digested with BamHI and extracted with phenol and then with chloroform, followed by ethanol precipitation to obtain a vector. 0.5 µg of the obtained vector was ligated with the above-obtained fragment having BamHI sites on its both ends and containing about 670 bp coding for TNF using $T_4$ DNA ligase. In accordance with the procedure described in Literature (3), page 250, E. coli JM103 (sold by Bethesda Research Laboratories, Inc., U.S.A.) was transformed using the above-obtained vector and cultivated on an agar medium containing 1 mM of IPTG and 0.004% (w/v) of x-gal to obtain about 200 white colonies. Plasmid DNA was prepared from 100 to these transformants and digested with BamHI. As a result, it was found that 15 plasmids contained the intended

14

BamHI fragment (about 670 bp). In order to examine the direction of insertion, the 15 plasmids are digested with EcoRI having only one recognition site on its pUC-8 and PvuII having only one recognition site on its about 670 base pair fragment part and electrophoresed on a 6% by weight polyacrylamide gel. As a result, it was affirmed that 7 plasmids had the intended fragment consisting of about 140 bp and that the direction of transcription of the lac promoter on pUC-8 is in agreement with that of the oligodeoxyribonucleotides coding for TNF.

DNA sequence analysis showed that these seven plasmids had the same sequence and had the desired nucleotide sequence at the junctions between the lac promoter, synthetic DNA and cDNA. The transformant containing the plasmid is designated as *E. coli* K-12 strain JM103 (pTNF-lac-1) (ATCC accession number 39677).

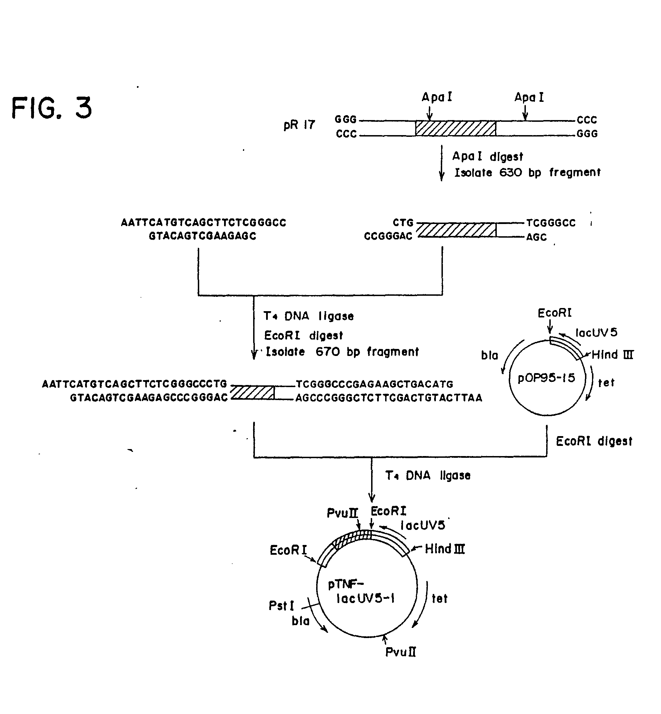

Construction of plasmids was carried out using the recombinant plasmid pR12 in order to obtain direct expression of TNF in *E. coli* using lac UV5 as a promoter. The procedures are illustratively shown in Fig. 3. First, 10 µg of the plasmid pR12 was digested with 10 units of ApaI (manufactured and sold by Bethesda Research Laboratories, Inc, U.S.A.) at 37°C for two hours and electrophoresed on a 4% by weight polyacrylamide gel to isolate a fragment consisting of about 630 bp. About 1 µg of the fragment was isolated from the gel by electroelution. In the same manner as in Step 8, two oligodeoxynucleotides shown in Fig. 3, namely 5'-AATTCATGTCAGCTTCTCGGGCC-3' and 5'-CGAGAAGCTGACATG-3' were synthesized. Then, each 5' end of the two oligodeoxynucleotides (about 100 pmole) was phosphorylated using $T_4$ polynucleotide kinase in accordance with the method described in Literature (3), page 122. After completion of the reaction, the reaction mixture was extracted with phenol and then with chloroform. Then the synthetic oligomers were mixed with 0.5 µg of the previously obtained ApaI fragment (about 630 bp) prepared from the plasmid pR12 and ethanol precipitated. The fragment was ligated with the synthetic oligomers at 4°C overnight using 10 units of $T_4$ ligase in accordance with procedure described in Literature (1), page 37. After completion of the reaction, the reaction mixture was ethanol precipitated and digested with 20 units of EcoRI at 37°C for three hours, followed by electrophoresis effected on a 4% by weight polyacrylamide gel to recover a fragment (about 670 bp) by electroelution.

In accordance with the procedure described in F. Fuller, "Gene", *19*, pp 42—54 (1982), plasmid pOP95-15 was prepared.

One µg of pOP95-15 was digested with EcoRI and extracted with phenol and then with chloroform, followed by ethanol precipitation to obtain a vector. Using $T_4$ DNA ligase, 0.5 µg of the obtained vector was ligated with the fragment (about 670 bp) obtained by ligating the synthetic oligonucleotide with the oligonucleotide coding for TNF. In accordance with the procedure described in Literature (3), page 250, *E. coli* JM103 was transformed using the above-obtained vector and cultivated on a medium containing 1 mM of IPTG and 0.004% (w/v) of x-gal to otbain about 150 white colonies. Plasmid DNA was prepared from 100 of these colonies and digested with EcoRI. As a result, it was found that 12 plasmids contained the intended EcoRI fragment (about 670 bp). In order to examine the direction of insertion, the above 12 plasmids were digested with PvuII and PstI and electrophoresed on a 1.5% by weight agarose gel. As a result, it was affirmed that four plasmids has the desired fragments (about 1280 bp and about 2600 bp) and that the direction of transcription of the lac UV5 promoter was in agreement with that of the oligodeoxynucleotides coding for TNF.

Base sequence analysis showed that these four plasmids had the same sequence and that the lac UV5 promoter, the synthetic oligodeoxynucleotide and cDNA were properly combined each other. The obtained plasmids were designated pTNF-lacUV5-1.

Step 12
(Purification of TNF produced by *E. coli*)

*E. coli* strains containing plasmids obtained in Step 11 were cultivated on 50 ml of LB medium containing 100 µg/ml of ampicillin at 37°C overnight. Then the strains were transferred to 5 liters of LB medium containing 100 µg/ml of ampicillin and further cultivated at 37°C for three hours. Isopropyl-β-D-thiogalactopyranoside (manufactured and sold by Sigma Chemical Company, Inc., U.S.A.) was added to it to a final concentration of 1 mM. Further cultivation was carried out for six hours, followed by cooling. Then strains were collected by centrifugation. In the same manner as described in Step 11, the strains were added into 5 liters of 0.04 M Tris-HCl buffer solution (pH 7.8) and disruption by sonication to obtain a strain protein solution. The obtained solution had cytotoxic activity against L cells of $6.7 \times 10^7$ units/liter.

The obtained solution was purified in the same manner as in Step 2 of Example 1 to obtain $1.2 \times 10^6$ units of TNF. The specific activity of the TNF was $6.8 \times 10^7$ units/mg.

Step 13
(Evaluation using transplanted Meth A sarcoma in mouse)

$2 \times 10^5$ Meth A sarcoma cells were transplanted intradermally at abdomen of a BALB/c mouse and, 7 days later, mice with tumors of 7 to 8 mm in diameter and with no spontaneous central necrosis were selected for evaluation. A sample (0.2 ml) of TNF obtained in Step 12 of Example 12 and diluted with physiological saline solution was injected through the tail vein. The activity of the sample was evaluated after 24 hours according to the following criteria.

(−)     no change

(+)     slight hemorrhagic necrosis

(++)    moderate hemorrhagic necrosis (central necrosis extending over approximately 50% of the tumor surface)

(+++) marked hemorrhagic necrosis (massive necrosis leaving a small viable rim along the tumor periphery)

20 days after the injection of the sample, observations were made on the involution of tumors and the recovery rate was determined according to the following equation.

$$\text{Recovery rate} = \frac{\text{Number of mice which had been completely recovered from tumor}}{\text{Number of mice used for test}}$$

The results are shown in Table 3.

## TABLE 3

| Injected amount of rabbit TNF produced by *E. coli* units/mouse | Number of mice used for test | Evaluation for activity of samples (after 1 day) | | | | Recovery rate (after 20 days) |
|---|---|---|---|---|---|---|
| | | − | + | ++ | +++ | |
| $2 \times 10^5$ | 5 | 0 | 0 | 1 | 4 | 5/5 |
| Reference (physiological saline) | 5 | 5 | 0 | 0 | 0 | 0/5 |

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

**Claims**

1. A physiologically active polypeptide having an amino acid sequence represented by the following formula (I):

```
Ser Ala Ser Arg Ala Leu Ser Asp Lys Pro Leu Ala His Val Val
Ala Asn Pro Gln Val Glu Gly Gln Leu Gln Trp Leu Ser Gln Arg
Ala Asn Ala Leu Leu Ala Asn Gly Met Lys Leu Thr Asp Asn Gln
Leu Val Val Pro Ala Asp Gly Leu Tyr Leu Ile Tyr Ser Gln Val
Leu Phe Ser Gly Gln Gly Cys Arg Ser Tyr Val Leu Leu Thr His
Thr Val Ser Arg Phe Ala Val Ser Tyr Pro Asn Lys Val Asn Leu
Leu Ser Ala Ile Lys Ser Pro Cys His Arg Glu Thr Pro Glu Glu
Ala Glu Pro Met Ala Trp Tyr Glu Pro Ile Tyr Leu Gly Gly Val
Phe Gln Leu Glu Lys Gly Asp Arg Leu Ser Thr Glu Val Asn Gln
Pro Glu Tyr Leu Asp Leu Ala Glu Ser Gly Gln Val Tyr Phe Gly
Ile Ile Ala Leu
```

wherein Gln stands for a glutamine residue, Asp an aspartic acid residue, Pro a proline residue, Tyr a tyrosine residue, Val a valine residue, Lys a lysine residue, Glu a glutamic acid residue, Ala an alanine residue, Asn an asparagine residue, Leu a leucine residue, Phe a phenylalanine residue, Gly a glycine residue, His a histidine residue, Ser a serine residue, Thr a threonine residue, Ile an isoleucine residue, Trp a tryprophan residue, Arg an arginine residue, Met a methionine residue, and Cys a cysteine residue.

2. A deoxyribonucleic acid comprising a base sequence coding for a physiologically active polypeptide, said physiologically active polypeptide having an amino acid sequence represented by the following formula (I):

```
Ser Ala Ser Arg Ala Leu Ser Asp Lys Pro Leu Ala His Val Val
Ala Asn Pro Gln Val Glu Gly Gln Leu Gln Trp Leu Ser Gln Arg
Ala Asn Ala Leu Leu Ala Asn Gly Met Lys Leu Thr Asp Asn Gln
```

```
Leu  Val  Val  Pro  Ala  Asp  Gly  Leu  Tyr  Leu  Ile  Tyr  Ser  Gln  Val
Leu  Phe  Ser  Gly  Gln  Gly  Cys  Arg  Ser  Tyr  Val  Leu  Leu  Thr  His
Thr  Val  Ser  Arg  Phe  Ala  Val  Ser  Tyr  Pro  Asn  Lys  Val  Asn  Leu
Leu  Ser  Ala  Ile  Lys  Ser  Pro  Cys  His  Arg  Glu  Thr  Pro  Glu  Glu
Ala  Glu  Pro  Met  Ala  Trp  Tyr  Glu  Pro  Ile  Tyr  Leu  Gly  Gly  Val
Phe  Gln  Leu  Glu  Lys  Gly  Asp  Arg  Leu  Ser  Thr  Glu  Val  Asn  Gln
Pro  Glu  Tyr  Leu  Asp  Leu  Ala  Glu  Ser  Gly  Gln  Val  Tyr  Phe  Gly
Ile  Ile  Ala  Leu
```

wherein Gln stands for a glutamine residue, Asp an aspartic acid residue, Pro a proline residue, Tyr a tyrosine residue, Val a valine residue, Lys a lysine residue, Glu a glutamic acid residue, Ala an alanine residue, Asn an asparagine residue, Leu a leucine residue, Phe a phenylalanine residue, Gly a glycine residue, His a histidine residue, Ser a serine residue, Thr a threonine residue, Ile an isoleucine residue, Trp a tyrptophan residue, Arg an arginine residue, Met a methionine residue, and Cys a cysteine residue.

3. A deoxyribonucleic acid comprising at least one base sequence selected from the group consisting of a base sequence represented by the following formula (II) and a complementary base sequence to said base sequence:

```
TCA GCT TCT CGG GCC CTG AGT GAC AAG CCT CTA GCC CAC GTA GTA
GCA AAC CCG CAA GTG GAG GGC CAG CTC CAG TGG CTG AGC CAG CGT
GCG AAC GCC CTG CTG GCC AAC GGC ATG AAG CTC ACG GAC AAC CAG
CTG GTG GTG CCG GCC GAC GGG CTG TAC CTC ATC TAC TCC CAG GTT
CTC TTC AGC GGT CAA GGC TGC CGC TCC TAC GTG CTC CTC ACT CAC
ACT GTC AGC CGC TTC GCC GTC TCC TAC CCG AAC AAG GTC AAC CTC
CTC TCT GCC ATC AAG AGC CCC TGC CAC CGG GAG ACC CCC GAG GAG
GCT GAG CCC ATG GCC TGG TAC GAG CCC ATC TAC CTG GGC GGC GTC
TTC CAG TTG GAG AAG GGT GAC CGG CTC AGC ACC GAG GTC AAC CAG
CCT GAG TAC CTG GAC CTT GCC GAG TCC GGG CAG GTC TAC TTT GGG
ATC ATT GCC CTG
```

wherein A stands for a deoxyadenylic acid residue, G a deoxyguanylic acid residue, C a deoxycytidylic acid residue and T thymidylic acid residue and wherein the left end and right end of the formula (II) represent 5'-hydroxyl group side and 3'-hydroxyl group side, respectively.

4. A deoxyribonucleic acid comprising a base sequence which is one obtained by substituting at least one base of said at least one base sequence of the deoxyribonucleic acid of claim 3 in accordance with degeneracy of genetic code.

5. A replicable recombinant DNA which comprises a deoxyribonucleic acid according to any one of claims 2 to 4 and a replicable expression vehicle.

6. A microorganism or cell culture transformed with a replicable recombinant DNA according to claim 5.

7. A method for producing a physiologically active polypeptide according to claim 1 which comprises:

(a) ligating a deoxyribonucleic acid according to any one of claims 2 to 4 to a replicable expression vehicle to obtain a replicable recombinant DNA comprising said deoxyribonucleic acid and said replicable expression vehicle;

(b) transforming cells of a microorganism or cell culture with said replicable recombinant DNA to form transformants;

(c) selecting said transformants from parent cells of the microorganism or cell culture;

(d) incubating said transformants, causing said transformants to express said deoxyribonucleic acid and produce a physiologically active polypeptide; and

(e) isolating said physiologically active polypeptide from the incubated transformants.

8. A substantially pure physiologically active polypeptide having the amino acid sequence as defined in claim 1.

9. A substantially pure deoxyribonucleic acid comprising a base sequence coding for a polypeptide having the amino acid sequence as defined in Claim 1.

10. A substantially pure deoxyribonucleic acid comprising the sequence as defined in claim 3.

11. A plasmid or bacteriophage transfer vector comprising a base sequence coding for a physiologically active polypeptide as defined in claim 1.

12. A microorganism or cell culture transformed by the transfer vector of claim 11, and mutants or variants thereof.

13. The microorganism E. coli K-12 strain JM103 (pTNF-lac-1).

14. The microorganism E. coli K-12 strain JM103 (pTNF-lacUV5-1).

15. The plasmid pTNF-lac-1.

16. The plasmid pTNF-lacUV5-1.

17

17. A pharmaceutical composition comprising an effective antitumor or antiviral amount of a physiologically active polypeptide as defined in claim 8 and at least one pharmaceutically acceptable carrier, diluent or excipient.

18. A pharmaceutical composition comprising an effective antitumor or antiviral amount of a physiologically active polypeptide as defined in claim 1 and at least one pharmaceutically acceptable carrier, diluent or excipient.

**Patentansprüche**

1. Physiologisch aktives Polypeptid mit einer Aminosäuresequenz der folgenden Formel (I):

```
Ser  Ala  Ser  Arg  Ala  Leu  Ser  Asp  Lys  Pro  Leu  Ala  His  Val  Val
Ala  Asn  Pro  Gln  Val  Glu  Gly  Gln  Leu  Gln  Trp  Leu  Ser  Gln  Arg
Ala  Asn  Ala  Leu  Leu  Ala  Asn  Gly  Met  Lys  Leu  Thr  Asp  Asn  Gln
Leu  Val  Val  Pro  Ala  Asp  Gly  Leu  Tyr  Leu  Ile  Tyr  Ser  Gln  Val
Leu  Phe  Ser  Gly  Gln  Gly  Cys  Arg  Ser  Tyr  Val  Leu  Leu  Thr  His
Thr  Val  Ser  Arg  Phe  Ala  Val  Ser  Tyr  Pro  Asn  Lys  Val  Asn  Leu
Leu  Ser  Ala  Ile  Lys  Ser  Pro  Cys  His  Arg  Glu  Thr  Pro  Glu  Glu
Ala  Glu  Pro  Met  Ala  Trp  Tyr  Glu  Pro  Ile  Tyr  Leu  Gly  Gly  Val
Phe  Gln  Leu  Glu  Lys  Gly  Asp  Arg  Leu  Ser  Thr  Glu · Val  Asn  Gln
Pro  Glu  Tyr  Leu  Asp  Leu  Ala  Glu  Ser  Gly  Gln  Val  Tyr  Phe  Gly
Ile  Ile  Ala  Leu
```

worin Gln einen Glutaminrest, Asp einen Asparaginsäurerest, Pro einen Prolinrest, Tyr einen Tyrosinrest, Val einen Valinrest, Lys einen Lysinrest, Glu einen Glutaminosäurerest, Ala einen Alaninrest, Asn einen Asparaginrest, Leu einen Leucinrest, Phe einen Phenylalaninrest, Gly einen Glycinrest, His einen Histidinrest, Ser einen Serinrest, Thr einen Threoninrest, Ile einen Isoleucinrest, Trp einen Tryptophanrest, Arg einen Argininrest, Met einen Methioninrest und Cys einen Cysteinrest bedeutet.

2. Deoxyribonukleinsäure mit einer Basensequenz, die ein physiologisch aktives Polypeptid kodiert, wobei das physiologisch aktive Polypeptid eine Aminosäuresequenz der folgenden Formel (I) besitzt:

```
Ser  Ala  Ser  Arg  Ala  Leu  Ser  Asp  Lys  Pro  Leu  Ala  His  Val  Val
Ala  Asn  Pro  Gln  Val  Glu  Gly  Gln  Leu  Gln  Trp  Leu  Ser  Gln  Arg
Ala  Asn  Ala  Leu  Leu  Ala  Asn  Gly  Met  Lys  Leu  Thr  Asp  Asn  Gln
Leu  Val  Val  Pro  Ala  Asp  Gly  Leu  Tyr  Leu  Ile  Tyr  Ser  Gln  Val
Leu  Phe  Ser  Gly  Gln  Gly  Cys  Arg  Ser  Tyr  Val  Leu  Leu  Thr  His
Thr  Val  Ser  Arg  Phe  Ala  Val  Ser  Tyr  Pro  Asn  Lys  Val  Asn  Leu
Leu  Ser  Ala  Ile  Lys  Ser  Pro  Cys  His  Arg  Glu  Thr  Pro  Glu  Glu
Ala  Glu  Pro  Met  Ala  Trp  Tyr  Glu  Pro  Ile  Tyr  Leu  Gly  Gly  Val
Phe  Gln  Leu  Glu  Lys  Gly  Asp  Arg  Leu  Ser  Thr  Glu  Val  Asn  Gln
Pro  Glu  Tyr  Leu  Asp  Leu  Ala  Glu  Ser  Gly  Gln  Val  Tyr  Phe  Gly
Ile  Ile  Ala  Leu
```

worin Gln einen Glutaminrest, Asp einen Asparaginsäurerest, Pro einen Prolinrest, Tyr einen Tyrosinrest, Val einen Valinrest, Lys einen Lysinrest, Glu einen Glutaminsäurerest, Ala einen Alaninrest, Asn einen Asparaginrest, Leu einen Leucinrest, Phe einen Phenylalaninrest, Gly einen Glycinrest, His einen Histidinrest, Ser einen Serinrest, Thr einen Threoninrest, Ile einen Isoleucinrest, Trp einen Tryptophanrest, Arg einen Argininrest, Met einen Methioninrest und Cys einen Cysteinrest bedeutet.

3. Deoxyribonukleinsäure mit mindestens einer Basensequenz aus der Gruppe, die aus der Basensequenz der folgenden Formel (II) und einer zu dieser Basensequence komplementären Basensequenz besteht:

```
TCA  GCT  TCT  CGG  GCC  CTG  AGT  GAC  AAG  CCT  CTA  GCC  CAC  GTA  GTA
GCA  AAC  CCG  CAA  GTG  GAG  GGC  CAG  CTC  CAG  TGG  CTG  AGC  CAG  CGT
GCG  AAC  GCC  CTG  CTG  GCC  AAC  GGC  ATG  AAG  CTC  ACG  GAC  AAC  CAG
CTG  GTG  GTG  CCG  GCC  GAC  GGG  CTG  TAC  CTC  ATC  TAC  TCC  CAG  GTT
CTC  TTC  AGC  GGT  CAA  GGC  TGC  CGC  TCC  TAC  GTG  CTC  CTC  ACT  CAC
ACT  GTC  AGC  CGC  TTC  GCC  GTC  TCC  TAC  CCG  AAC  AAG  GTC  AAC  CTC
CTC  TCT  GCC  ATC  AAG  AGC  CCC  TGC  CAC  CGG  GAG  ACC  CCC  GAG  GAG
GCT  GAG  CCC  ATG  GCC  TGG  TAC  GAG  CCC  ATC  TAC  CTG  GGC  GGC  GTC
TTC  CAG  TTG  GAG  AAG  GGT  GAC  CGG  CTC  AGC  ACC  GAG  GTC  AAC  CAG
CCT  GAG  TAC  CTG  GAC  CTT  GCC  GAG  TCC  GGG  CAG  GTC  TAC  TTT  GGG
ATC  ATT  GCC  CTG
```

worin A einen Deoxyadenylsäurerest, G einen Deoxyguanylsäurerest, C einen Deoxycytidylsäurerest und T einen Thymidylsäurerest bedeuten und worin das linke Ende und das rechte Ende der Formel (II) die 5'-hydroxylgruppen-Seite bzw. die 3'-Hydroxylgruppen-Seite repräsentieren.

4. Deoxyribonukleinsäure mit einer Basensequenz, die dadurch erhalten worden ist, daß man mindestens eine Base mindestens einer Basensequenz der Deoxyribonukleinsäure gemäß Anspruch 3 gemäß einer Abwandlung des genetischen Codes subtituiert.

5. Replizierbare, rekombinante DNA mit einer Deoxyribonukleinsäure gemäß einem der Ansprüche 2 bis 4 und einem replizierbaren Expressionsvehikel.

6. Mikroorganismus oder Zellkultur, die mit einer replizierbaren, rekombinanten DNA gemäß Anspruch 5 transformiert worden sind.

7. Verfahren zur Herstellung eines physiologisch aktiven Polypeptids gemäß Anspruch 1, bei dem man

(a) eine Deoxyribonukleinsäure gemäß einem der Ansprüche 2 bis 4 mit einem replizierbaren Expressionsvehikel verknüpft, um eine replizierbare, rekombinante DNA mit der Deoxyribonukleinsäure und dem replizierbaren Expressionsvehikel zu erhalten,

(b) Zellen eines Mikroorganismus oder einer Zellkultur mit der replizierbaren, rekombinanten DNA zur Bildung von Transformanten transformiert,

(c) die Transformanten von Vorgängerzellen des Mikroorganismus oder der Zellkultur selektiert,

(d) die Transformanten inkubiert, so daß die Transformanten die Deoxyribonukleinsäure exprimieren und ein physiologisch aktives Polypeptid liefern, und

(e) das physiologisch aktive Polypeptid von den inkubierten Transformanten isoliert.

8. Im wesentlichen reines physiologisch aktives Polypeptid mit der Aminosäuresequenz gemäß Anspruch 1.

9. Im wesentlichen reine Deoxyribonukleinsäure mit einer Basensequenz, die ein Polypeptid mit der Aminosäuresequenz gemäß Anspruch 1 kodiert.

10. Im wesentlichen reine Deoxyribonukleinsäure mit der Sequenz gemäß Anspruch 3.

11. Plasmid oder Bakteriophagentransfervektor mit einer Basensequenz, die ein physiologisch aktives Polypeptid gemäß Anspruch 1 kodiert.

12. Mikroorganismus oder Zellkultur, die durch den Transfervektor gemäß Anspruch 11 transformiert sind, und Mutanten und varianten davon.

13. Mikroorganismus E.-coli-K-12-Stamm JM103 (pTNF-lac-1).

14. Mikroorganismus E.-coli-K-12-Stamm JM103 (pTNF-lacUV5-1).

15. Plasmid pTNF-lac-1.

16. Plasmid pTNF-lacUV5-1.

17. Pharmazeutische Zusammensetzung mit einer effektiven antitumoralen oder antiviralen Menge eines physiologisch aktiven Polypeptide gemäß Anspruch 8 und mindestens einem pharmazeutisch verträglichen Träger, Verdünnungsmittel oder Streckmittel.

18. Pharmazeutische Zusammensetzung mit einer effektiven antitumoralen oder antiviralen Menge eines physiologisch aktiven Polypeptids gemäß Anspruch 1 und mindestens einem pharmazeutisch verträglichen Träger, Verdünnungsmittel oder Streckmittel.


**Revendications**

1. Polypeptide physiologiquement actif ayant une séquence d'acides aminés représentée par la formule (I) qui suit:

| | | | | | | | | | | | | | | |
|---|---|---|---|---|---|---|---|---|---|---|---|---|---|---|
| Ser | Ala | Ser | Arg | Ala | Leu | Ser | Asp | Lys | Pro | Leu | Ala | His | Val | Val |
| Ala | Asn | Pro | Gln | Val | Glu | Gly | Gln | Leu | Gln | Trp | Leu | Ser | Gln | Arg |
| Ala | Asn | Ala | Leu | Leu | Ala | Asn | Gly | Met | Lys | Leu | Thr | Asp | Asn | Gln |
| Leu | Val | Val | Pro | Ala | Asp | Gly | Leu | Tyr | Leu | Ile | Tyr | Ser | Gln | Val |
| Leu | Phe | Ser | Gly | Gln | Gly | Cys | Arg | Ser | Tyr | Val | Leu | Leu | Thr | His |
| Thr | Val | Ser | Arg | Phe | Ala | Val | Ser | Tyr | Pro | Asn | Lys | Val | Asn | Leu |
| Leu | Ser | Ala | Ile | Lys | Ser | Pro | Cys | His | Arg | Glu | Thr | Pro | Glu | Glu |
| Ala | Glu | Pro | Met | Ala | Trp | Tyr | Glu | Pro | Ile | Tyr | Leu | Gly | Gly | Val |
| Phe | Gln | Leu | Glu | Lys | Gly | Asp | Arg | Leu | Ser | Thr | Glu | Val | Asn | Gln |
| Pro | Glu | Tyr | Leu | Asp | Leu | Ala | Glu | Ser | Gly | Gln | Val | Tyr | Phe | Gly |
| Ile | Ile | Ala | Leu | | | | | | | | | | | |

où Gln indique un résidu de glutamine, Asp un résidu d'acide aspartique, Pro un résidu de proline, Tyr un résidu de tyrosine, Val un résidu de valine, Lys un résidu de lysine, Glu un résidu d'acide glutamique, Ala un résidu d'alanine, Asn un résidu d'asparagine, Leu un résidu de leucine, Phe un résidu de phénylalanine, Gly un résidu de glycine, His un résidu d'histidine, Ser un résidu de sérine, Thr un résidu de thréonine, Ile un résidu d'isoleucine, Trp un résidu de tryptophane, Arg un résidu d'arginine, Met un résidu de méthionine et Cys un résidu de cystéine.

2. Acide désoxyribonucléique comprenant une séquence de bases codant pour un polypeptide physiologiquement actif,

ledit polypéptide physiologiquement actif ayant une séquence d'acides aminés représentée par la formule (I) qui suit:

| | | | | | | | | | | | | | | |
|---|---|---|---|---|---|---|---|---|---|---|---|---|---|---|
| Ser | Ala | Ser | Arg | Ala | Leu | Ser | Asp | Lys | Pro | Leu | Ala | His | Val | Val |
| Ala | Asn | Pro | Gln | Val | Glu | Gly | Gln | Leu | Gln | Trp | Leu | Ser | Gln | Arg |
| Ala | Asn | Ala | Leu | Leu | Ala | Asn | Gly | Met | Lys | Leu | Thr | Asp | Asn | Gln |
| Leu | Val | Val | Pro | Ala | Asp | Gly | Leu | Tyr | Leu | Ile | Tyr | Ser | Gln | Val |
| Leu | Phe | Ser | Gly | Gln | Gly | Cys | Arg | Ser | Tyr | Val | Leu | Leu | Thr | His |
| Thr | Val | Ser | Arg | Phe | Ala | Val | Ser | Tyr | Pro | Asn | Lys | Val | Asn | Leu |
| Leu | Ser | Ala | Ile | Lys | Ser | Pro | Cys | His | Arg | Glu | Thr | Pro | Glu | Glu |
| Ala | Glu | Pro | Met | Ala | Trp | Tyr | Glu | Pro | Ile | Tyr | Leu | Gly | Gly | Val |
| Phe | Gln | Leu | Glu | Lys | Gly | Asp | Arg | Leu | Ser | Thr | Glu | Val | Asn | Gln |
| Pro | Glu | Tyr | Leu | Asp | Leu | Ala | Glu | Ser | Gly | Gln | Val | Tyr | Phe | Gly |
| Ile | Ile | Ala | Leu | | | | | | | | | | | |

où Gln indique un résidu de glutamine, Asp un résidu d'acide aspartique, Pro un résidu de proline, Tyr un résidu de tyrosine, Val un résidu de valine, Lys un résidu de lysine, Glu un résidu d'acide glutamique, Ala un résidu d'alanine, Asn un résidu d'asparagine, Leu un résidu de leucine, Phe un résidu de phénylalanine, Gly un résidu de glycine, His un résidu d'histidine, Ser un résidu de sérine, Thr un résidu de thréonine, Ile un résidu d'isoleucine, Trp un résidu de tryptophane, Arg un résidu d'arginine, Met un résidu de méthionine et Cys un résidu de cystéine.

3. Acide désoxyribonucléique comprenant au moins une séquence de bases choisie dans le groupe consistant en une séquence de bases représentée par la formule (II) qui suit, et une séquence de bases complémentaire de ladite séquence de bases:

```
TCA GCT TCT CGG GCC CTG AGT GAC AAG CCT CTA GCC CAC GTA GTA
GCA AAC CCG CAA GTG GAG GGC CAG CTC CAG TGG CTG AGC CAG CGT
GCG AAC GCC CTG CTG GCC AAC GGC ATG AAG CTC ACG GAC AAC CAG
CTG GTG GTG CCG GCC GAC GGG CTG TAC CTC ATC TAC TCC CAG GTT
CTC TTC AGC GGT CAA GGC TGC CGC TCC TAC GTG CTC CTC ACT CAC
ACT GTC AGC CGC TTC GCC GTC TCC TAC CCG AAC AAG GTC AAC CTC
CTC TCT GCC ATC AAG AGC CCC TGC CAC CGG GAG ACC CCC GAG GAG
GCT GAG CCC ATG GCC TGG TAC GAG CCC ATC TAC CTG GGC GGC GTC
TTC CAG TTG GAG AAG GGT GAC CGG CTC AGC ACC GAG GTC AAC CAG
CCT GAG TAC CTG GAC CTT GCC GAG TCC GGG CAG GTC TAC TTT GGG
ATC ATT GCC CTG
```

dans laquelle A indique un résidu d'acide désoxyadénylique, G un résidu d'acide désoxyguanylique, C un résidu d'acide désoxycytidylique et T un résidu d'acide thymidylique et où l'extrémité gauche et l'extrémité droite de la formule (II) représentent le côté du groupe 5'-hydroxyle et le côté du groupe 3'-hydroxyle, respectivement.

4. Acide désoxyribonucléique comprenant une séquence de bases qui est celle obtenue par substitution d'au moins une base de ladite au moins une séquence de bases de l'acide désoxyribonucléique de la revendication 3 selon la dégénérescence du code génétique.

5. ADN recombinant réplicable qui comprend un acide désoxyribonucléique selon l'une quelconque des revendications 2 à 4 et un vecteur d'expression réplicable.

6. Microorganisme ou culture de cellules transformé par un ADN recombinant réplicable selon la revendication 5.

7. Méthode de production d'un polypeptide physiologiquement actif selon la revendication 1 qui comprend:

(a) la ligature d'un acide désoxyribonucléique selon l'une quelconque des revendications 2 à 4 à un vecteur d'expression réplicable pour obtenir un ADN recombinant réplicable comprenant ledit acide désoxyribonucléique et ledit vecteur d'expression réplicable;

(b) la transformation des cellules d'une microorganisme ou d'une culture de cellules par ledit ADN recombinant réplicable pour former des transformants;

(c) la sélection desdits transformants de cellules parentes du microorganisme ou de la culture de cellules;

(d) l'incubation desdits transformants, forçant lesdits transformants à exprimer ledit acide désoxyribonucléique et à produire un polypeptide physiologiquement actif; et

(e) l'isolement dudit polypeptide physiologiquement actif des transformants incubés.

8. Polypeptide actif sensiblement physiologiquement pur ayant la séquence d'acides aminés telle que définie à la revendication 1.

9. Acide désoxyribonucléique sensiblement pur comprenant une séquence de bases codant pour un polypeptide ayant la séquence d'acides aminés telle que définie à la revendication 1.

10. Acide désoxyribonucléique sensiblement pur comprenant la séquence telle que définie à la revendication 3.

11. Vecteur plasmidique ou de transfert bactériophage comprenant une séquence de bases codant pour un polypeptide physiologiquement actif selon la revendication 1.

12. Microorganisme ou culture de cellules transformé par le vecteur de transfert de la revendication 11 et ses mutants ou variants.

13. Microorganisme E. coli K-12 souche JM103 (pTNF-lac-1).

14. Microorganisme E. coli K-12 souche JM103 (pTNF-lacUV5-1).

15. Plasmide pTNF-lac-1.

16. Plasmide pTNF-lacUV5-1.

17. Composition pharmaceutique comprenant une quantité efficace antitumorale ou antivirale d'un polypeptide physiologiquement actif tel que défini à la revendication 8 et d'au moins un véhicule, diluant ou excipient acceptable en pharmacie.

18. Composition pharmaceutique comprenant une quantité antitumorale ou antivirale efficace d'un polypeptide physiologiquement actif tel que défini à la revendication 1 et au moins un véhicule, diluant ou excipient acceptable en pharmacie.

FIG. I

# FIG. 2

# FIG. 3